# EUROPÄISCHE PATENTSCHRIFT

(11) **EP 1 246 909 B1**
(45) Veröffentlichungstag und Bekanntmachung des Hinweises auf die Patenterteilung: **22.03.2006**
(21) Anmeldenummer: 00991270.0
(22) Anmeldetag: 27.12.2000
(51) Int. Cl.: C12N 9/88, C12N 1/21, C12P 21/02

(54) **VERFAHREN ZUR HERSTELLUNG VON HYDROXYNITRIL-LYASEN**
METHOD FOR THE PRODUCTION OF HYDROXYNITRILE LYASES
PROCEDE DE FABRICATION D'HYDROXYNITRILE-LYASES

(30) Priorität: 28.12.1999 DE 19963485
(43) Veröffentlichungstag der Anmeldung: 09.10.2002
(73) Patentinhaber: Julich Chiral Solutions GmbH, 52428 Jülich (DE)
(72) Erfinder: EFFENBERGER, Franz, 70569 Stuttgart (DE); FÖRSTER, Siegfried, 70569 Stuttgart (DE); WAJANT, Harald, 70771 Leinfelden (DE)
(74) Vertreter: Schrell, Andreas
(86) Internationale Anmeldenummer: PCT/EP2000/013280
(87) Internationale Veröffentlichungsnummer: WO 2001/048178

(56) Entgegenhaltungen:
- BREITHAUPT, H. ET AL.: "Cloning and expression of (R)-hydroxynitrile lyase from Linum usitatissimum (flax)" JOURNAL OF MOLECULAR CATALYSIS B: ENZYMATIC, Bd. 6, Nr. 3, 11. März 1999 (1999-03-11), Seiten 315-332, XP000995739
- HASSLACHER, M. ET AL.: "High-level intracellular expression of hydroxynitrile lyase from the tropical rubber tree Hevea brasiliensis in microbial hosts" PROTEIN EXPRESSION AND PURIFICATION, Bd. 11, Nr. 1, 1997, Seiten 61-71, XP000915456 in der Anmeldung erwähnt
- WAJANT, H. UND PFIZENMAIER, K.: "Identification of potential active-site residues in the hydroxynitrile lyase from Manihot esculenta by site-directed mutagenesis" JOURNAL OF BIOLOGICAL CHEMISTRY, Bd. 271, Nr. 42, 18. Oktober 1996 (1996-10-18), Seiten 25830-25834, XP000995344 in der Anmeldung erwähnt
- HUANG, K.-X. ET AL.: "Overproduction, in Escherichia coli, of soluble taxadiene synthase, a key enzyme in the taxol biosynthetic pathway" PROTEIN EXPRESSION AND PURIFICATION, Bd. 13, Juni 1998 (1998-06), Seiten 90-96, XP000996132
- WAJANT, H. ET AL.: "Molecular cloning of hydroxynitrile lyase from Sorghum bicolor (L.). Homologies to serine carboxypeptidases" PLANT MOLECULAR BIOLOGY, Bd. 26, Nr. 2, 1994, Seiten 735-746, XP000995778

## Beschreibung

Die vorliegende Erfindung betrifft ein Verfahren zur Herstellung von Hydroxynitril-Lyasen.

Cyanhydrine, also α-Hydroxynitrile, werden durch basen-katalysierte Addition von Cyanwasserstoff an Carbonylverbindungen wie aliphatische, alicyclische, ungesättigte, aromatisch substituierte aliphatische, aromatische oder heteroaromatische Aldehyde und Ketone gebildet. Dabei entstehen bei prochiralen Carbonylverbindungen racemische Cyanhydrine. Cyanhydrine können zur Synthese von α-Hydroxysäuren, α-Hydroxyketonen, β-Aminoalkoholen oder ähnlichem dienen, die wiederum für die Herstellung biologisch wirksamer Stoffe, z.B. pharmazeutischer Wirkstoffe, Vitamine oder im Pflanzenschutz verwendbarer Verbindungen, eingesetzt werden können. Insbesondere mittels enzymatischer Verfahren ist es möglich, optisch aktive (R)- oder (S)-Cyanhydrine in hoher optischer Reinheit zu erhalten. So beschreibt die EP-A-0 326 063 ein enzymatisches Verfahren zur Herstellung von optisch aktiven (R)- oder (S)-Cyanhydrinen durch Umsetzung von aliphatischen, aromatischen oder heteroaromatischen Aldehyden und Ketonen mit Cyanwasserstoff in Gegenwart von (R)-Hydroxynitril-Lyase aus Prunus amygdalis (EC 4.1.2.10) oder von (S)-Hydroxynitril-Lyase aus Sorghum bicolor (EC 4.1.2.11).

Aus der EP-A-0 632 130 geht ein Verfahren hervor, gemäß dem aliphatische Aldehyde oder unsymmetrische aliphatische Ketone mit Cyanwasserstoff und Hydroxynitril-Lyase aus Hevea brasiliensis stereospezifisch zu (S)-Cyanhydrinen umgesetzt werden.

Hydroxynitril-Lyasen (HNLS) wurden aus einer Reihe unterschiedlicher Pflanzenarten isoliert. Hydroxynitril-Lyasen lassen sich in zwei Klassen einteilen, wobei Hydroxynitril-Lyasen der Klasse I aus Pflanzen der Familie Rosaceae stammen, Cofaktorabhängig sind und eine Molekularmasse von 50 bis 80 kDa aufweisen (z.B. HNL aus Prunus sp.). Hydroxynitril-Lyasen der Klasse II stammen aus Pflanzen der Familie Olacaceae, Gramineae, Linaceae und Euphorbiaceae, sind Cofaktor-unabhängig und weisen Molekularmassen von 28 bis 42 kDa auf. Bekannte Hydroxynitril-Lyasen dieser Klasse stammen aus Sorghum bicolor (SbHNL), Manihot esculenta (MeHNL), Hevea brasiliensis (HbHNL) oder Linum usitatissimum (LuHNL).

Zur Herstellung möglichst großer und reiner Mengen von Hydroxynitril-Lyasen ist es wünschenswert, diese nicht aus natürlichen Quellen zu isolieren, sondern gentechnisch herzustellen, zum Beispiel durch heterologe Expression in Saccharomyces cerevisiae-, Pichia pastoris- oder E. coli-Zellen, die mittels Vektoren transformiert wurden, welche ein Hydroxynitril-Lyasen-codierendes und exprimierbares Gen aufweisen.

Aus Hasslacher et al. (J. Biol. Chem. 271 (1996), 5884-5891) geht hervor, dass die heterologe Expression größerer Mengen von HbHNL in E. coli mit dem Problem der Bildung von unlöslichen Einschlusskörpern, sogenannten inclusion bodies, behaftet ist. Da solche Einschlusskörper in aller Regel kein funktionsfähiges Protein enthalten, müssen derartige Einschlusskörper in zusätzlichen Verfahrensschritten renaturiert werden, um eine Konformationsänderung des Proteins in die aktive Form zu ermöglichen. Auch aus Hasslacher et al. (Protein Expression and Purification 11 (1997), 61-71) und Trummler et al. (Plant Science 139 (1998), 19-27) geht hervor, dass die heterologe Expression von HbHNL bzw. LuHNL in E. coli zwar zu der Bildung größerer Mengen an HNL führt, diese jedoch in unlöslicher Form ohne signifikante Aktivität vorliegt. E. coli-Zellen scheinen demgemäss nicht dafür geeignet zu sein, die heterologe Genexpression von HNL in nativer und aktiver Konformation zu gewährleisten. Breithaupt et al. (Journal of Molecular Catalysis B, Enzymatic 6 (3), 315-332 (1999)) offenbart Verfahren zur Expression von HNL aus Linum usitatissimus in E. coli.

Die Etablierung eines auch für den technischen Maßstab einsetzbaren Expressionssystems wird zudem durch das Erfordernis erschwert, dass sowohl unterschiedliche natürlicherweise vorkommende, also Wildtyp-Hydroxynitril-Lyasen aus verschiedenen Spezies mit unterschiedlichen Substratspezifitäten als auch Mutanten dieser Wildtyp-Hydroxynitril-Lyasen in möglichst einem einheitlichen System mit hoher Verlässlichkeit, großer Effizienz und hoher Ausbeute gebildet werden sollen.

So sind aus Wajant und Pfizenmaier (J. Biol. Chem. 271 (1996), 25830-25834) verschiedene Mutanten der MeHNL bekannt. Die mittels des dort beschriebenen Verfahrens gebildete Menge des Wildtyp- und mutanten Enzyms ist jedoch nicht für den industriellen Einsatz ausreichend.

Das der vorliegenden Erfindung zugrundeliegende technische Problem besteht also darin, ein Verfahren zur Herstellung von Hydroxynitril-Lyasen, insbesondere (S)-Hydroxynitril-Lyasen bereitzustellen, das unabhängig von Struktur, insbesondere Aminosäuresequenz, und Herkunft der zu bildenden Hydroxynitril-Lyase diese in möglichst großer Ausbeute und Reinheit herzustellen vermag.

Die Erfindung löst das ihr zugrundeliegende Problem durch die Bereitstellung eines Verfahrens zur Herstellung einer Hydroxynitril-Lyase (auch HNL genannt) in gelöster Form, wobei Bakterien der Art Escherichia coli enthaltend mindestens ein HNL-codierendes Gen in einem Kulturmedium bei 30°C bis 40°C vorkultiviert, mit 1 bis 100 *µ*M, vorzugsweise 1 bis 50 *µ*M, insbesondere 1 bis 30 *µ*M und besonders bevorzugt 1 bis 20 *µ*M IPTG (Endkonzentration in Kulturmedium) induziert, bei 15 bis 25°C kultiviert und aufgeschlossen werden sowie anschließend gelöste HNL gewonnen wird.

Das erfindungsgemäße Verfahren zeichnet sich in überraschender Weise dadurch aus, dass es die Bildung von HNL in großer Menge in gelöster nativer und aktiver Form, also nicht in Form von inclusion bodies, ermöglicht. Zeit- und materialaufwendige sowie Aktivitätsverlust bedeutende Renaturierungsverfahren sind erfindungsgemäß also nicht mehr notwendig. Die Erfindung weist ferner den Vorteil auf, dass mittels des erfindungsgemäßen Verfahrens nicht nur die Wildtyp-Enzyme, sondern auch ihre mutanten Derivate in gleicher Effizienz ohne Rücksicht auf deren Herkunft, Struktur, insbesondere Aminosäuresequenz oder Konformation in löslicher und aktiver Form erhalten werden können. Die erfindungsgemäße Verfahrensweise ermöglicht es also erstmals, mittels eines einzigen Systems eine Vielzahl unterschiedlicher HNLs und HNL-Mutanten so zu exprimieren, dass diese in technischem Maßstab gewonnen werden können und ggf. direkt ohne weitere chemische oder physikalische Aufarbeitung für die Cyanhydrin-Herstellung eingesetzt werden können.

Im Zusammenhang mit der vorliegenden Erfindung werden unter Hydroxynitril-Lyasen Enzyme verstanden, die die Addition von Cyanwasserstoff an die Carbonylgruppe eines Aldehyds oder Ketons katalysieren. Die Erfindung erfasst sowohl (R)-HNLs als auch (S)-HNLs. (R)-Hydroxynitril-Lyasen sind z.B. LuHNL, PhaHNL (Phlebodium aureum) oder HNLs aus Prunus sp., z.B. Prunus amygdalis oder Prunus serotina (PsHNL). (S)-HNLs sind z.B. SbHNL, MeHNL und HbHNL.

Im Zusammenhang mit der vorliegenden Erfindung werden unter dem Begriff Hydroxynitril-Lyasen nicht nur die nativen, d.h. die Wildtyp-Enzyme verstanden, sondern auch durch Mutagenese erzeugte Varianten dieser Enzyme, d.h. zum Beispiel aus gentechnisch modifizierten Mikroorganismen gewonnene Hydroxynitril-Lyasen. Diese können entweder die Wildtyp-Sequenz oder eine davon abgewandelte Aminosäuresequenz aufweisen. Derartige mutante Abwandlungen weisen also z.B. aufgrund von Aminosäureadditionen, -deletionen, -inversionen oder -austauschen eine geänderte Aminosäuresequenz gegenüber dem Wildtyp-Enzym auf. Derartige mutante Hydroxynitril-Lyasen können verkürzte Hydroxynitril-Lyasen oder durch Fusion mit anderen Peptiden oder Proteinen verlängerte Hydroxynitril-Lyasen sein. Erfindungsgemäß können die Hydroxynitril-Lyasen auch Signal-, Export- oder Erkennungspeptide aufweisen, die z.B. einen Export in den periplasmatischen oder extrazellulären Raum des die HNLbildenden Bakteriums bewirken. Erfindungsgemäß ist es möglich, dass die gebildete Hydroxynitril-Lyase zunächst in Translationseinheit mit einem weiteren Peptid oder Protein hergestellt wird und erst nach der Expression und ggf. Sekretion eine Spaltung des Translationsproduktes in die Hydroxynitril-Lyase und einen Restbestandteil erfolgt. Selbstverständlich können die gebildeten Hydroxynitril-Lyasen auch Modifikationen anderer Art aufweisen, wie Derivatisierungen mit Zuckern, Lipiden, Glycolipiden oder Proteoglycanen. Selbstverständlich können die erfindungsgemäß hergestellten Hydroxynitril-Lyasen auch ungewöhnliche Aminosäuren aufweisen.

In besonders bevorzugter Ausführungsform ist die HNL eine HNL wie sie in Haslacher et al. 1996 und 1997, Wajant und Pfitzenmaier 1996 und Effenberger (Chimia 53 (1999), 3-10) beschrieben sind. Der Offenbarungsgehalt dieser Druckschriften ist hinsichtlich der Struktur, insbesondere Aminosäuresequenz, und Herstellung der dort beschriebenen Hydroxynitril-Lyasen in ihrer Wildtyp- und mutanten Form vollständig in den Offenbarungsgehalt der vorliegenden Lehre mit einbezogen.

Im Zusammenhang mit der vorliegenden Erfindung wird unter einem mindestens ein HNL-codierendes Gen enthaltenden Bakterium der Art E. coli ein E. coli-Bakterium verstanden, das einen Vektor, insbesondere ein Plasmid, vorzugsweise extrachromosomal, aufweist, welches zumindest ein in E. coli exprimierbares HNL-Gen aufweist. Dieses Gen weist die codierende Region der HNL sowie regulatorische Bereiche zur Expression desselben, z.B. einen Promoter und Terminationssignale, auf. Die codierenden Regionen codieren die vorstehend beschriebene Wildtyp- oder mutante Form der HNLs und weisen demgemäss ggf. Nucleotid-Insertionen, -deletionen, -austausche, -substitutionen oder die Insertion oder Addition ungewöhnlicher Nucleotide im Vergleich zur Wildtypnucleinsäuresequenz auf. Die regulatorischen Bereiche sind vorzugsweise Nucleinsäuresequenzen, die eine Expression der heterologen codierenden Region in E. coli ermöglichen.

Als Promoter kann beispielsweise der trc-Promoter oder der trp-lac-Promoter verwendet werden.

Das HNL-codierende Gen ist beispielsweise in dem Expressionsvektor pQE4 (Quiagen) enthalten. Beispiele für erfindungsgemäß einsetzbare Vektoren und HNL-DNAs für die erfindungsgemäße Expression in E. coli können Wajant und Pfizmaier 1996, Hasslacher et al. 1996 und 1997 entnommen werden, die hinsichtlich der dort beschriebenen E. coli-Expressionsvektoren sowie der beschriebenen Nucleotidseauenzen für Wildtyp- und mutante Formen der HNL vollständig in den Offenbarungsgehalt der vorliegenden Lehre mit einbezogen sind. Regulatorische Sequenzen, Vektoren, Verfahren zur Herstellung und Transformation der Vektoren sind z.B. aus Sambrook et al. (Molecular cloning: a laboratory manual. Cold Spring Harbor Laboratory Press, 1989).

Im Zusammenhang mit der vorliegenden Erfindung wird unter einem Kulturmedium jedes Medium verstanden, in dem Bakterien der Art E. coli leben, vorzugsweise sich vermehren können, insbesondere HNL bilden können.

In bevorzugter Ausführungsform der vorliegenden Erfindung wird die Kultur in TB-Medium (TB = Terrific Broth) oder synthetischem Medium durchgeführt, was zu einer besonders überraschend hohen Enzymausbeute führt. Das TB-Medium enthält in 900 ml deionisiertem Wasser 12 g Bacto-Trypton, 24 g Bacto-Hefeextrakt und 4 ml Glycerol, zu denen 100 ml einer sterilen Lösung von 0,17 M KH₂PO₄ und 0,72 M K₂HPO₄ gegeben wurden. Das synthetische Medium kann wie folgt zusammengesetzt sein: 2,0 g/l Na₂SO₄, 2,68 g/l (NH₄)₂SO₄, 0,5 g/l NH₄Cl, 14,6 g/l K₂HPO₄, 3,6 g/l NaP₂PO₄-H₂O, 1,0 g/l (NH₄)₂-H-citrat, 2,0 ml/l MgSO₄ (1 M), Spurenelemente (3,0 ml/l) (0,5 g CaCl₃·2H₂O, 0,18 g ZnSO₄-7H₂O, 0,10 g MnSO₄·H₂O, 20,1 g Na₂-EDTA, 16,7 g FeCl₃·6H₂O, 0,16 g CuSO₄·5H₂O, 0,18 g CoCl₃·6H₂O), 10,0 mg/l Thiamin, 10,0 g/l Glucose.

In einer weiteren bevorzugten Ausführungsform der vorliegenden Erfindung ist das Kulturmedium LB-Mediun (LB = Luria-Bertani), welches in 950 ml deionisierten Wasser 10 g Bacto-Trypton, 5 g Bacto-Hefeextrakt und 10 g NaCl enthält (pH 7,0, mit deionisiertem Wasser auf 1 l auffüllen).

Diese Medien werden vorzugsweise auch für die Vorkultivierung eingesetzt.

Die Erfindung betrifft ein vorgenanntes Verfahren, wobei die Kultivierung bei gegenüber Standardbedingungen reduzierten Temperaturen, nämlich 15 bis 25° C, vorzugsweise 20° C erfolgt. Dadurch ergibt sich eine besonders hohe Enzymausbeute.

Die Vorkultivierung wird bei 30 bis 40° C, insbesondere 37° C durchgeführt. Demgemäss wird das Kulturmedium nach erfolgter Vorkultivierung und Induktion abgekühlt und im abgekühlten Medium die Kultivierung und HNL-Expression durchgeführt.

Die Erfindung sieht in bevorzugter Ausführung weiterhin vor, dass die Bakterien nach der Kultivierung und vor dem Aufschluß vom Kulturmedium abgetrennt werden, insbesondere erfolgt die Abtrennung mittels Zentrifugation. Der sich daran anschließende Zellaufschluss wird vorteilhafterweise mittels Ultraschalleinwirkung durchgeführt, wobei jedoch auch andere Aufschlußverfahren z.B. mechanische Einwirkung oder Einfluß elektrischer Felder vorgesehen sein kann. Sofern die HNL in das Medium sezerniert wird, ist kein Zellaufschluss nötig. In einer solchen Ausgestaltung werden die Zellen vom Kulturmedium abgetrennt und das HNL-haltige Medium verwendet.

Die Gewinnung der so gebildeten und freigesetzten HNL aus dem Zellaufschluss wird z.B. wie in Wajant et al. (1994) Plant Science, 103, 145-154, Wajant et al. (1995) Plant Science, 108, 1-11, Wajant et al. (1996) Annals NY Acad. Sci., 799, 771-776 und Wajant und Förster (1996) Plant Science, 115, 25-31 beschrieben durch Gelausschlußchromatographie und Anionenaustauschchromatographie erreicht. Die vorgenannten Offenbarungen sind hinsichtlich der Gewinnung von HNL vollständig in den vorliegenden Offenbarungsgehalt mit eingeschlossen.

Die Erfindung betrifft in einer weiteren Ausgestaltung mittels der vorgenannten Verfahren hergestellte Hydroxynitril-Lyase.

Weitere vorteilhafte Ausgestaltungen der Erfindung ergeben sich aus den Unteransprüchen.

Die Erfindung wird anhand der folgenden Beispielen näher erläutert.

### Beispiel 1:

Beschreibung der in den Beispielen 1 bis 3 verwendeten Kulturbedingungen.

Eine 4 1-Fermenterkultur (Infors) wird mit 40 ml einer 12 h alten Kultur von E. coli M15-MeHNL (Plasmid-transformierter 15-Stamm, enthält HNL aus Manihot esculenta vgl. Wajant und Pfizenmaier (1996)) bzw. E. coli JM 109-MeHNL in LB-Medium oder TB-Medium mit Ampicillin (100 mg/l) für den M15 bzw. JM 109-Stamm und Kanamycin (25 mg/l) nur für den M15-Stamm angeimpft. Nach ca. 12 h Vorkultivierung bei 37° C wird das Kulturmedium mit den Zellen abgekühlt und anschließend weitere 16 h bei der jeweils angegebenen Temperatur kultiviert. Die Expression der HNL wird dabei nach 4 h mit Isopropyl-β-D-thiogalactosid (IPTG) in der angegebenen Konzentration induziert. Zur Abtrennung vom Medium werden die Zellen 30 Minuten bei 6170 x g zentrifugiert. Das erhaltene Zellpellet wird mit 300 ml Natriumacetat-Puffer (20 mM, pH 5,4) resuspendiert und die Zellen durch Ultraschall 15 Minuten bei ca. 300 W aufgeschlossen. Die Enzymaktivität wird über die Bildung von Benzaldehyd aus 3,8 mM racemischem Mandelonitril in Testpuffer (50 mM Citratpuffer, pH 5,0) bestimmt. Die Zunahme der Benzaldehydabsorption bei 280 nm wird bei 25° C über 5 Minuten verfolgt (linearer Bereich des Tests).

Nach Abkühlung von 37° auf 30° C Kultivierungstemperatur und bei 1 mM IPTG-Endkonzentration in LB-Medium (Vergleichsbeispiel 1) wurden folgende in Tabelle 1 aufgeführten Daten ermittelt:

**Tabelle 1: Vergleichsbeispiel 1**

| Stamm | Feuchtzellmasse (g) | Gesamtaktivität (U) | Fermentervolumen (1) | Aktivität pro g Zellmasse (U/g) | Zellmasse pro l Fermenter (g/l) |
|---|---|---|---|---|---|
| MIS-MeHNL | 10 | 800 | 4 | 80 | 2,5 |
| JM 109-MeHNL | 25 | 2000 | 4 | 80 | 6,25 |

**Tabelle 2 Vergleichsbeispiel 2 (Bedingungen wie in Tabelle 1, aber Verwendung von synthetischem Medium)**

| Stamm | Feuchtzellmasse (g) | Gesamtaktivität (U) | Fermentervolumen (1) | Aktivität pro g Zellmasse (U/g) | Zellmasse pro l Fermenter (g/l) |
|---|---|---|---|---|---|
| M15-MeHNL | 23 | 1200 | 4 | 52 | 5,8 |
| JM 109-MeHNL | 20 | 1600 | 4 | 80 | 5 |

**Tabelle 3 Vergleichsbeispiel 3 (Bedingungen wie in Tabelle 1, aber Verwendung von TB-Medium)**

| Stamm | Feuchtzellmasse (g) | Gesamtaktivität (U) | Fermentervolumen (1) | Aktivität pro g Zellmasse (U/g) | Zellmasse pro l Fermenter (g/1) |
|---|---|---|---|---|---|
| M15-MeHNL | 18 | 1230 | 4 | 68 | 4,5 |
| JM 109-MeHNL | 36 | 2430 | 4 | 67,5 | 9 |

Nach Abkühlung von 37° auf 20° C Kultivierungstemperatur und bei 100 µM IPTG-Endkonzentration in TB-Medium wurde folgendes Ergebnis erhalten:

**Tabelle 4**

| Stamm | Feuchtzellmasse (g) | Gesamtaktivität (U) | Fermentervolumen (1) | Aktivität pro g Zellmasse (U/g) | Zellmasse pro l Fermenter (g/l) |
|---|---|---|---|---|---|
| M15-MeHNL | 47 | 38000 | 4 | 808 | 11,7 |
| JM 109-MeHNL | 85 | 130000 | 4 | 1529 | 21,2 |

### Beispiel 2

Nach Abkühlung von 37° auf 20° C Kultivierungstemperatur und bei 100 *µ*M IPTG-Endkonzentration in TB-Medium wurde unter Einsatz von mutanten HNLs folgendes Ergebnis erhalten:

**Tabelle 5 (*Enzymaktivität für Mandelsäurehydroxynitril)**

| Stamm | Feuchtzellmasse (g) | Gesamtaktivität* (U) | Fermentervolumen (l) | Aktivität pro g Zellmasse (U/g) | Zellmasse pro l Fermenter (g/l) |
|---|---|---|---|---|---|
| M15-MeHNL-W128C | 110 | 184000 | 4 | 1673 | 27,5 |
| M15-MeHNL-W128V | 120 | 61500 | 4 | 512 | 30 |
| M15-MeHNL-W128Y | 112 | 284000 | 4 | 2536 | 28 |
| M15-MeHNL-C81A | 550 | 700000 | 25 | 1272 | 22 |
| M15-MeHNL-W128A | - 100 schleim ig | 64500 | 4 | 645 | 25 |
| M15-MeHNL-W128L | 58 | 134000 | 4 | 2310 | 14,5 |

### Beispiel 3:

Nach Abkühlung von 37°C auf 20°C Kultivierungstemperatur und bei 10 µM IPTG-Endkonzentration in TB-Medium wurde unter Einsatz von mutanten HNLs folgendes Ergebnis erhalten:

**Tabelle 6**

| Stamm | Feuchtzellmasse (g) | Gesamtaktivität (U) | Fermentervolumen (l) | Aktivität pro g Zellmasse (U/g) | Zellmasse pro l Fermenter (g/l) |
|---|---|---|---|---|---|
| JM 109-MeHNL | 30 | 6600 | 4 | 220 | 7,5 |

Die eingesetzten M15-Mutanten sind: In Position 128 der unverkürzten Aminosäuresequenz der MeHNL ist der Tryptophanrest durch Cystein (W128C), Valin (W128V), Tyrosin (W128Y) und Alanin (W128A) ersetzt. In C81A ist der Cysteinrest in Position 81 durch Alanin ersetzt.

Die M15-Mutanten werden in denselben bioaktiven Ausbeuten wie der Wildtyp erhalten.

## Patentansprüche

1. Verfahren zur Herstellung einer Hydroxynitril-Lyase in gelöster Form, wobei Bakterien der Art Escherichia coli enthaltend mindestens ein eine Hydroxynitril-Lyase codierendes Gen in einem Kulturmedium bei 30 bis 40°C vorkultiviert, mit 1 bis 100 µM IPTG (Endkonzentration im Kulturmedium) induziert, bei 15 bis 25°C kultiviert und aufgeschlossen werden sowie anschließend gelöste Hydroxynitril-Lyase gewonnen wird.

2. Verfahren nach Anspruch 1, wobei die Kultivierung bei 20° C erfolgt.

3. Verfahren nach Anspruch 1 oder 2, wobei die Vorkultivierung bei 37° C erfolgt.

4. Verfahren nach einem der vorhergehenden Ansprüche, wobei die Kultivierung in LB-Medium erfolgt.

5. Verfahren nach einem der Ansprüche 1 bis 4, wobei die Kultivierung in TB-Medium erfolgt.

6. Verfahren nach einem der vorhergehenden Ansprüche, wobei die Bakterien nach der Kultivierung und vor dem Aufschluß vom Kulturmedium abgetrennt werden.

7. Verfahren nach Anspruch 6, wobei die Abtrennung mittels Zentrifugation erfolgt.

8. verfahren nach einem der vorhergehenden Ansprüche, wobei der Zellaufschluss mittels Ultraschall erfolgt.

9. Verfahren nach einem der vorhergehenden Ansprüche, wobei das die Hydroxynitril-Lyase codierende Gen in den Bakterien auf einem Plasmid vorliegt.

10. Verfahren nach einem der vorhergehenden Ansprüche, wobei die Hydroxynitril-Lyase eine natürlich vorkommende Hydroxynitril-Lyase oder eine mutante Abwandlung davon ist.

11. Verfahren nach einem der vorhergehenden Ansprüche, wobei die Hydroxynitril-Lyase aus Sorghum bicolor, Manihot esculenta, Hevea brasiliensis, Linum usitatissimum oder Prunus sp. stammt.

## Claims

1. Method for the production of a hydroxynitrile lyase in dissolved form, whereby bacteria of the Escherichia coli type containing at least one hydroxynitrile lyase-coding gene are pre-cultured in a culture medium at 30 to 40°C, induced with 1 to 100 µM IPTG (final concentration in the culture medium), cultured at 15 to 25°C, and lysed, and subsequently dissolved hydroxynitrile lyase is obtained.

2. Method according to claim 1, whereby the culturing is carried out at 20°C.

3. Method according to claim 1 or 2, whereby the pre-culturing is carried out at 37°C.

4. Method according to any one of the preceding claims, whereby the culturing is carried out in LB medium.

5. Method according to any one of the claims 1 to 4, whereby the culturing is carried out in TB medium.

6. Method according to any one of the preceding claims, whereby the bacteria are separated from the culture medium after culturing and prior to lysis.

7. Method according to claim 6, whereby the separation is carried out by means of centrifugation.

8. Method according to any one of the preceding claims, whereby the cell lysis is carried out by means of ultrasound.

9. Method according to any one of the preceding claims, whereby the gene coding for the hydroxynitrile lyase is present on a plasmid in the bacteria.

10. Method according to any one of the preceding claims, whereby the hydroxynitrile lyase is a naturally occurring hydroxynitrile lyase or a mutant modification thereof.

11. Method according to any one of the preceding claims, whereby the hydroxynitrile lyase originates from Sorghum bicolor, Manihot esculenta, Hevea brasiliensis, Linum usitatissimum or Prunus sp.

## Revendications

1. Procédé de fabrication d'une hydroxynitrile-lyase sous forme dissolue, des bactéries de type *Escherichia coli* contenant au moins un gène codant une hydroxynitrile-lyase étant précultivées dans un milieu de culture à une température allant de 30°C à 40°C, induites par 1 à 100 µM IPTG (concentration finale dans le milieu de culture), cultivées à une température allant de 15°C à 25°C et désagrégées, et une hydroxynitrile-lyase dissolue étant ensuite obtenue.

2. Procédé selon la revendication 1, la culture ayant lieu à 20°C.

3. Procédé selon la revendication 1 ou 2, la préculture ayant lieu à 37°C.

4. Procédé selon l'une des revendications précédentes, la culture ayant lieu dans un milieu LB.

5. Procédé selon l'une des revendications 1 à 4, la culture ayant lieu dans un milieu TB.

6. Procédé selon l'une des revendications précédentes, les bactéries étant séparées après la culture et avant la désagrégation du milieu de culture.

7. Procédé selon la revendication 6, la séparation se produisant par centrifugation.

8. Procédé selon l'une des revendications précédentes, la désagrégation des cellules se produisant au moyen d'ultrasons.

9. Procédé selon l'une des revendications précédentes, le gène codant l'hydroxynitrile-lyase dans les bactéries étant présent sur un plasmide.

10. Procédé selon l'une des revendications précédentes, l'hydroxynitrile-lyase étant une hydroxynitrile-lyase naturelle ou une modification mutante de celle-ci.

11. Procédé selon l'une des revendications précédentes, l'hydroxynitrile-lyase provenant de *Sorghum bicolor,* de *Manihot esculenta, Hevea brasiliensis, Linum usitatissimum* ou *Prunus sp.*
